Europäisches Patentamt

European Patent Office

Office européen des brevets

(11) Numéro de publication: **0 308 330**
**A1**

(12) **DEMANDE DE BREVET EUROPEEN**

(21) Numéro de dépôt: **88402322.7**

(22) Date de dépôt: **15.09.88**

(51) Int. Cl.⁴: **C 12 N 11/10**

(30) Priorité: **17.09.87 FR 8712886**

(43) Date de publication de la demande:
**22.03.89 Bulletin 89/12**

(84) Etats contractants désignés:
**BE DE FR GB IT NL**

(71) Demandeur: **SANOFI**
**40, Avenue George V**
**F-75008 Paris (FR)**

(72) Inventeur: **Schacht, Etienne**
**Rijsseveldstraat 99**
**BE-8140 Staden (BE)**

**Nobels, Mia**
**Laurent Delvaux Straat 4**
**BE-9000 Gand (BE)**

(74) Mandataire: **Gillard, Marie-Louise**
**Cabinet Beau de Loménie 55, Rue d'Amsterdam**
**F-75008 Paris (FR)**

(54) **Protéines, enzymes, ou micro-organismes immobilisés sur un support à base de gélatine réticulée par un polysaccharide oxydé.**

㊼ Les produits sont immobilisés par réticulation d'un mélange du produit considéré, de gélatine et d'un polysaccharide oxydé tel qu'un xanthane oxydé ou un dextran oxydé.

Les compositions peuvent se présenter sous forme de particules, de grains, de films. Application aux réactions enzymatiques, procédés de fermentation ou dosages immunologiques.

EP 0 308 330 A1

Bundesdruckerei Berlin

# Description

## Protéines, enzymes, ou micro-organismes immobilisés sur un support à base de gélatine réticulée par un polysaccharide oxydé.

La présente invention concerne l'immobilisation de protéines, d'enzymes ou de micro-organismes sur un support à base de gélatine insolubilisée par réticulation avec un polysaccharide oxydé, tel qu'un dextran oxydé ou un xanthane oxydé.

L'immobilisation d'enzymes, ou de micro-organismes, lors de la mise en oeuvre de réactions enzymatiques, que ce soit sur de grandes quantités de matières comme lors de fermentations conduisant à des produits industriels variés ou à petite échelle pour des procédés d'analyse et de dosage, est d'autant plus souhaitable qu'elle permet une séparation aisée des enzymes du milieu réactionnel et éventuellement leur recyclage.

On a proposé différents moyens d'immobilisation des enzymes dont notamment leur couplage à l'aide de réactifs bifonctionnels à des polymères insolubles. On peut par exemple citer l'immobilisation de la lactase soit dans des fibres de triacétate de cellulose, décrite par F. MORISI dans J. of DAIRY SCIENCE 56(9) p. 1123-27 (1973), soit dans un polyacrylamide, décrite par K. OHMIYA dans APPLIED AND ENVIRONMENTAL MICROBIOL. 33(1) p. 137-146 (1977).

Il a aussi été décrit l'immobilisation d'enzymes dans un support à base de gélatine par action de dialdéhydes aliphatiques, de dichlorures d'acides et autres réactifs difonctionnels analogues, dans les brevets US 3838007 et GB 1455993 ou par action de formaldéhyde dans le brevet DE 2636206. On a aussi décrit l'immobilisation de levures, par réticulation des grains obtenus par émulsification d'une solution aqueuse de gélatine et de levures et gélification dans EP 0222462 tandis que, dans le brevet FR 2035774, a été décrit l'immobilisation de protéines ayant une activité thérapeutique sur une protéine inerte, comme l'albumine, par action d'un réactif bifonctionnel, comme le glutaraldéhyde.

Un des problèmes majeurs rencontrés lors de l'immobilisation des enzymes est la diminution de leur activité et les méthodes précédemment citées ne l'ont que partiellement résolu; il faut en effet que les protéines ou les microorganismes ne soient pas dénaturés dans les conditions d'immobilisation, et qu'ils restent accessibles à leurs substrats bien que solidement fixés à leurs supports.

On a maintenant trouvé que l'on pouvait avantageusement immobiliser, avec un excellent rendement, des enzymes et autres protéines ou des microorganismes en faisant réagir sur un mélange de gélatine et du produit à immobiliser, un polysaccharide convenablement choisi, qui a été préalablement oxydé de telle sorte que des fonctions aldéhydes réactives aient été créées à partir de certaines de ses fonctions alcools.

Le polysaccharide approprié aux fins de l'invention peut être notamment un dextran oxydé ou un xanthane oxydé.

Les polysaccharides, tels que les alginates, dans lesquels ils se forment, par oxydation periodique, des fonctions hémiacétals stables, qui ne réagissent pas sur les fonctions amine des protéines, ne sont pas utilisables selon l'invention. D'autre part, les polysaccharides faciles à oxyder, tels que l'amidon et les pectines ne réagissent sur la gélatine que lentement et à pH élevé, incompatible avec la plupart des protéines et des microorganismes. Ils ne sont donc pas non plus utilisables selon l'invention.

Les compositions selon l'invention peuvent se présenter sous forme de particules ou de grains, de films, de fibres ou être imprégnées sur des supports poreux, comme des papiers-filtres ou des tissus, selon l'application envisagée. Les grains sphériques qui, humides, ont un diamètre compris entre 0,5 et 3 mm, seront préférés pour une utilisation en continu, en colonne, mais peuvent être avantageusement utilisés aussi en réacteurs, tout comme les particules obtenues par concassage ou broyage et tamisage d'une masse réticulée.

On sait qu'il existe deux types de gélatine : celle de TYPE A ou acide, de point isoélectrique voisin de 8, qui est préparée par hydrolyse acide du collagène des os ou des peaux de porcins et de bovins, et celle de TYPE B de point isoélectrique voisin de 5, qui est préparée par hydrolyse basique du même collagène.

Les deux types de gélatine peuvent être utilisés selon l'invention, en adaptant les conditions opératoires de telle sorte que la réaction avec le polysaccharide oxydé ait lieu dans un milieu dont le pH est supérieur au point isoélectrique de la gélatine; on préfère une gélatine de type A, ce qui permet d'effectuer la réticulation à un pH voisin de la neutralité entre 6 et 8 et une gélatine de bloom élevé pur avoir des gels réticulés plus rigides.

Le polysaccharide oxydé mis en oeuvre selon l'invention ne doit pas être très épaississant et gélifiant, notamment pour permettre son oxydation en solution aux concentrations habituelles; en outre, le polysaccharide oxydé doit être suffisament soluble en milieu aqueux pour former une phase homogène concentrée avec la solution aqueuse de gélatine contenant les protéines ou les microorganismes à immobiliser pour obtenir une masse suffisamment rigide en quelques heures.

On préfère un polysaccharide de masse moléculaire inférieure à 500000 et même entre 10000 et 100000, de telle sorte que les viscosités des solutions aqueuses du polysaccharide ne soient pas trop élevées, par exemple de 0,1 à 1 Pa.s pour une solution à 2 % étudiée au viscosimètre Brookfield LVT (30 tours). Pour le xanthane, on utilisera en général des produits résultants de la dépolymérisation du polysaccharide natif par hydrolyse selon des procédés classiques.

L'oxydation des polysaccharides est une réaction connue, qui a notamment été utilisée pour obtenir des produits servant à la réticulation de gélatines entrant dans la composition de films photographiques.

On sait qu'elle peut être réalisée, en milieu aqueux, par action d'un sel d'acide periodique, comme le periodate de sodium, comme décrit dans le brevet belge n⁰ 566 352, les brevets anglais n⁰ 891221 et 907625, le brevet allemand n⁰ 1073305 ou le brevet américain n⁰ 3062652.

On peut aussi l'effectuer dans un solvant organique tel que le diméthylsulfoxyde, par action de l'acide periodique ou du tétraacétate de plomb.

La vitesse de réticulation de la gélatine est fonction, notamment, de la masse moléculaire du polysaccharide oxydé, de sa concentration dans le milieu, et du nombre de groupes aldéhydes qu'il porte ainsi que du pH. Le spécialiste déterminera par des moyens classiques quel est le nombre d'unités devant être oxydées sur la chaîne du polysaccharide, en fonction des conditions opératoires envisagées pour la réticulation et donc la quantité d'oxydant à utiliser.

Pour le dextran et le xanthane sur lesquels deux fonctions aldéhydes sont créées par unité osidique par action d'une ou deux molécules de periodate, on a constaté que, pour la préparation de grains d'enzymes immobilisées à pH sensiblement neutre, le pourcentage d'unités oxydées devrait être de préférence compris entre 10 et 75 % et mieux entre 20 et 50 %. Dans ces conditions, on peut fait réagir le polysaccharide et la gélatine en quantités équivalentes, en solution aqueuse, à température ambiante, de concentration comprise entre 2 et 10 % environ.

Les polysaccharides, convenablement oxydés, peuvent être isolés par des méthodes classiques ; par exemple, on peut les faire précipiter dans un milieu aqueux, par addition d'un tiers solvant, tel que l'acétone, le méthanol ou l'isopropanol ou lyophiliser la solution d'oxydation après dialyse.

Les polysaccharides séchés sont stables et sont introduits au moment de l'emploi dans le milieu contenant la gélatine à réticuler et le produit à immobiliser.

Dans la présente description, on entend par enzyme à immobiliser, aussi bien une enzyme pure, qu'un mélange d'enzymes, qui sont associés ou non à d'autres constituants cellulaires ou même les micro-organismes les contenant. On peut immobiliser selon l'invention de nombreuses enzymes et micro-organismes ; toutefois, les enzymes protéolytiques et les osidases qui réagiraient sur leur support, ainsi que les enzymes qui se fixeraient sur le support par l'intermédiaire d'une fonction présente dans leur site actif, sont exclues.

Parmi les enzymes immobilisables, on peut citer, sans que l'invention y soit limitée, l'amyloglucosidase utilisée dans la fabrication de la bière et des sirops de glucose, la tannase pour la clarification du vin et de la bière, la β-galactosidase ou lactase pour l'hydrolyse de petit-lait, la pectinase pour l'industrie des jus de fruits et légumes, la pénicilline acylase utilisée en hémi-synthèse d'antibiotiques, la glucose-isomérase pour l'obtention de sirop de maïs à fort taux de fructose, l'uréase pour le dosage de l'urée, la lipase pour la formation de savons et l'industrie fromagère, l'invertase pour l'hydrolyse du saccharose, les acylase, décarboxylase, transaminase et racémase pour la fabrication d'aminoacides.

Des micro-organismes, bactéries, levures ou champignons peuvent aussi être immobilisés. On peut citer l'espèce bactérienne Escherichia coli, utilisée après modification génétique pour la synthèse de nombreuses protéines, l'espèce bactérienne Lactobacillus bulgaricus pour la fermentation du lait, le genre bactérien Xanthomonas producteur de xanthane, les champignons de type Penicillium utilisés pour la synthèse d'antibiotiques, les bactéries de l'espèce Leuconostoc mesenteroïdes qui transforment le saccharose en fructose et glucose-1 phosphate, les levures Saccharomyces cerevisiae pour la fabrication d'éthanol ou les champignons Mortiella vinacea pour le raffinage du sucre de betterave.

On sait qu'il est avantageux d'immobiliser d'autres types de protéines et notamment des antigènes et des anticorps pour la réalisation de dosages immunologiques, mettant en oeuvre des principes bien connus du spécialiste, notamment la méthode ELISA, et ces protéines immobilisées sont un autre objet de l'invention.

On prépare les compositions selon l'invention, en introduisant, à un pH supérieur au point isoélectrique de la gélatine, le polysaccharide oxydé dans une solution aqueuse de gélatine contenant les enzymes, les micro-organismes ou les protéines à immobiliser.

La vitesse de la réaction de réticulation est fonction du polysaccharide oxydé utilisé, mais aussi du milieu de réticulation et notamment du pH ; le pH devra être d'autant élevé que le degré d'oxydation est plus faible et on choisira de préférence un degré d'oxydation voisin de 50 % si la protéine ou l'enzyme sont dénaturées à pH élevé. Ainsi, on a constaté qu'un dextran oxydé à 45 % (45 % des unités glucose oxydées) réagissait sur la gélatine rapidement à pH 6,5, tandis qu'avec un dextran oxydé à 20 % il était préférable d'opérer à pH 8.

Par contre, on notera qu'à pH 7,7 un amidon oxydé à 50 % ne donne aucune réticulation et ce polysaccharide ne peut donc pas convenir à la préparation de compositions selon l'invention; avec un tel polysaccharide, il faut se placer à pH 10 pour obtenir une réticulation comme pour la caroube, et les pectines, pH incompatible avec la plupart des produits à immobiliser selon l'invention.

On peut utiliser par exemple de 20 % à 60 % en poids de polysaccharide oxydé par rapport à la gélatine, et de préférence le même poids de gélatine et de polysaccharide oxydé : la vitesse de réticulation croît avec la quantité de polysaccharide et son degré d'oxydation.

La quantité de produit à immobiliser dans la composition sera en fonction de son activité intrinsèque et de l'application envisagée ; pour que la composition ait une bonne tenue mécanique, le produit immobilisé constituera, en général, moins de 10 % en poids de la composition.

Les compositions de l'invention sont insolubles en milieu aqueux, ne fondent pas lorsqu'elles sont portées à 60°C, et leurs propriétés mécaniques permettent de les utiliser en colonne, sous forme de grains de diamètre compris entre 0,5 et 3 mm, pour la mise en oeuvre de procédés de synthèse enzymatique et de fermentation.

Les compositions peuvent être isolées et conservées à l'état sec, ou en suspension aqueuse, de préférence en présence d'un agent conservateur comme l'azoture de sodium et éventuellement en enceinte réfrigérée. Après séchage, elles peuvent être regonflées partiellement dans l'eau.

Pour préparer les compositions de l'invention sous forme de film, on étale la solution aqueuse tamponnée fraîchement préparée contenant la gélatine, le produit à immobiliser et le polysaccharide oxydé, à une température supérieure à celle de gélification du milieu, en général supérieure à 30°C, et inférieure à la température de dénaturation de l'enzyme, du micro-organisme ou de la protéine en utilisant des moyens d'étalement classiques pour fixer l'épaisseur de la couche, et on laisse reposer jusqu'à la fin de la réaction de réticulation.

Pour préparer des fibres, on utilise la technique connue d'extrusion de la masse gélifiée constituée de gélatine, polysaccharide oxydé et produit à immobiliser, avant qu'elle ne soit totalement réticulée.

Pour préparer des particules, on peut réticuler totalement la solution aqueuse du mélange de départ et diviser ensuite la masse obtenue en fines particules par découpage, broyage, opérations suivies de préférence d'un tamisage.

Pour préparer des grains sphériques ou sensiblement sphériques, on peut opérer par atomisation, mais on préfère introduire la solution aqueuse des 3 constituants, divisée sous forme de gouttelettes, dans une solution d'un solvant organique non miscible à l'eau ou émulsionner cette solution dans un tel solvant, dans des conditions telles que la gélification de la paroi se produise rapidement et laisser ensuite les grains en suspension durcir spontanément dans le milieu où ils ont été formés ou, de préférence, dans un milieu aqueux tamponné, dans lequel on peut, par exemple, les introduire par gravité.

Parmi les solvants organiques non miscibles à l'eau, on préférera les liquides aprotiques, comme les hydrocarbures aliphatiques ou aromatiques ou les huiles, sur lesquels les fonctions aldéhydes ne peuvent réagir. Lorsque l'on opère par gravité, on introduit, par exemple, en haut d'une colonne remplie d'un solvant organique non miscible à l'eau et terminée par un récipient collecteur qui contient une phase aqueuse tamponnée où s'achèvera la réticulation, les gouttelettes du mélange à réticuler ; la hauteur de la colonne de solvant sera déterminée par le spécialiste de telle sorte qu'à l'arrivée de la gouttelette dans la phase aqueuse sa paroi soit suffisamment durcie pour qu'elle ne soit plus déformable, et le solvant choisi de telle sorte que sa densité soit inférieure à celle de l'eau.

Comme solvants organiques convenant particulièrement bien à la réalisation de l'invention, on peut citer l'éther de pétrole, la décaline, le cyclohexane, le benzène, l'huile de paraffine ou une huile de silicone.

La durée de la réaction de réticulation dépend de la quantité et de la nature du polysaccharide oxydé, du milieu de réticulation ainsi que de la forme et des dimensions unitaires de la composition ; il faut en général de 1 à 24 h avant que les compositions enzymatiques de l'invention puissent être isolées du milieu où elles ont été préparées, lavées et séchées s'il y a lieu.

Dans ce qui suit, on décrit des exemples de réalisation de l'invention.

### EXEMPLE 1

### β-galactosidase immobilisée dans une composition de gélatine réticulée avec du dextran oxydé.

### I - Préparation d'une composition sous forme de grains

a) Oxydation du polysaccharide.

On dissout 20 g de dextran de masse moléculaire moyenne 70 000, commercialisé par Pharmacia sous la référence T70, dans 200 ml de tampon phosphate à pH 6 (force ionique 0,2). On ajoute dans cette solution, sous agitation, une solution aqueuse de periodate de sodium préparée à partir de 7,92 g de periodate et 20 ml d'eau. Après deux heures à température ambiante, on isole le polysaccharide oxydé du milieu réactionnel, par précipitation par un tiers solvant. Avant précipitation, on peut éliminer l'iodate formé, en traitant cette solution par 30 g d'iodure de potassium en solution dans 150 ml d'acide chlorhydrique dilué (0,05 M), avant de verser le mélange dans 500 ml de méthanol pour précipiter le dextran oxydé.

On peut aussi dialyser le milieu réactionnel contre l'eau pure et, lorsque tout le periodate a été éliminé, concentrer la solution pour isoler le polysaccharide.

On a déterminé, par titrimétrie à l'hydroxylamine, que 20 % des fonctions alcools secondaires du dextran étaient oxydées.

b) Immobilisation de l'enzyme.

On dissout, d'une part, 0,3 g de gélatine de point isoélectrique 5 et de Bloom 200 (mesuré par la méthode AOAC) et, d'autre part, 0,3 g du dextran oxydé précédemment obtenu, dans 3 ml de tampon phosphate à pH 8 en tiédissant légèrement.

On mélange les solutions et on ajoute 90 µl d'une solution de β-galactosidase contenant 55,3 unités/ml, avant d'introduire le mélange résultant à l'aide d'une seringue, sous forme de gouttelettes dans un récipient contenant une solution aqueuse tamponnée à pH 8 surmontée, sur une hauteur de 30 cm, de 1,5 l d'isooctane glacé et de laisser 20 h sous agitation.

La taille des gouttelettes est fonction du diamètre de l'aiguille utilisée pour l'introduction ; pour un diamètre de l'aiguille de 0,9 mm, on a obtenu 8 g de grains de diamètre 23 mm à l'état humide, ou 11 mm après séchage.

c) Isolement des grains.

Les grains réticulés sont isolés par filtration et séchés en dessiccateur en présence d'un déshydratant pendant 2 jours. Ils se rétractent en se déshydratant, mais reprennent leur forme lorsqu'ils sont remis en solution aqueuse.

## II - Etude des grains

### a) Importance et stabilité de l'immobilisation

On a vérifié qu'il n'y avait pas d'enzyme restant dans les milieux de réticulation et que, lorsque les grains sont laissés en solution aqueuse, il n'y a pas de libération de l'enzyme dans la solution : 8 g de grains isolés du milieu réactionnel, non séchés, sont introduits dans 10 ml de solution aqueuse tamponnée à pH 6,5.

Après 1 h d'agitation, on prélève 1 ml de cette solution et on l'introduit dans 2 ml d'une solution à 7,5 % (p/V) de lactose dans un tampon, dit "milk buffer", généralement utilisé pour la mesure de l'activité de la β-galactosidase et constitué de 0,795 g de citrate de Na, 1,662 g d'acide citrique, 0,18 g de sulfate de potassium, 0,52 g de monophosphate de potassium, 1,47 g de diphosphate de potassium, 1,09 g de potasse, 0,88 g de chlorure de magnésium, 0,75 g de chlorure de calcium, 0,4 g d'hydroxyde de sodium et 0,28 g de bicarbonate de sodium pour 1 l, et ajusté à pH 6,5 par addition de HCl.

Après 30 minutes à 37°C, on ajoute 1 ml d'acide trichloracétique et, après 15 minutes à température ambiante, on prélève 0,25 ml de cette solution et on l'introduit dans 5 ml de réactif pour le dosage du glucose, constitué du système enzymatique glucoseoxydase et peroxydase associé au chromogène : chlorhydrate d'Odianisidine commercialisé par Worthington Biochemical sous la marque Glucostat.

Après 50 minutes à 20°C, l'absorption est mesurée à 520 nm ; dans ces conditions, on ne met pas de glucose en évidence dans la solution prélevée, ce qui traduit l'absence de l'enzyme transformant le lactose en glucose et donc la stabilité de l'enzyme dans la composition.

### b) Activité de l'enzyme immobilisée dans les grains, comparée à celle de la même quantité d'enzyme en solution.

Les grains préparés comme précédemment contiennent 4,97 unités enzymatiques. Ils sont introduits dans 18 ml d'une solution de lactose à 7,5 % (p/V) dans le tampon précédemment décrit, et on ajoute 9 ml du même tampon, pour obtenir une solution contenant 0,183 unité enzymatique par ml. On prélève toutes les 15 minutes et pendant 2 heures un échantillon de ce mélange, maintenu à 37°C, et on mesure la quantité de glucose présent, en appliquant la méthode décrite dans l'essai mentionné en a), mais sans addition d'acide trichloracétique.

L'efficacité de l'immobilisation est égale au rapport des coefficients d'absorption mesurés dans le deux essais (enzyme immobilisée/enzyme en solution), multipliée par 0,75 pour tenir compte de la moindre dilution dans le cas des essais avec les compositions enzymatiques sous forme de grains.

Les résultats obtenus figurent dans le tableau I suivant :

| | Heure de prélèvement | Absorption | Efficacité |
|---|---|---|---|
| Enzyme en solution | 30 min | 0,415 | 100 % |
| Enzyme fixée | 30 min | 0,345 | 63 % |

On a constaté que l'efficacité de la composition enzymatique finale n'était pas modifiée si la réticulation était effectuée à pH 8 dans le tampon décrit au lieu d'être effectuée dans un tampon phosphate au même pH ; toutefois, la vitesse de réticulation est plus lente, compte tenu de la force ionique plus élevée du premier tampon.

On a obtenu des résultats équivalents en utilisant en dextran de masse moléculaire 40 000 environ, dont 45 % des fonctions alcools secondaires ont été oxydées ; la vitesse de réticulation étant plus rapide, on a dans ce cas effectué la réticulation dans le tampon "milk buffer" pH 6,5, au lieu du tampon phosphate.

### c) Efficacité de l'immobilisation dans la gélatine réticulée par le glutaraldéhyde, (exemple comparatif).

A titre de comparaison, on introduit 90 μl d'une solution aqueuse d'enzyme à 553 unités par ml dans une solution aqueuse à 8 % (p/V) de gélatine (Bloom = 200, point iso = 5).

Des gouttelettes de cette solution sont introduites dans un récipient contenant de l'acétate de butyle glacé. Les grains formés sont séparés après 60 minutes, lavés trois fois à l'éthanol et introduits dans une solution de glutaraldéhyde à 2,5 % (p/V). Après 3 heures, les grains durcis sont isolés et lavés à l'eau distillée. L'efficacité, déterminée comme précédemment, n'est que de 8 %.

## EXEMPLE 2

Leuconostoc mesenteroïdes immobilisés dans une composition réticulée avec du dextran oxydé

### I - Préparation d'une composition sous forme de grains.

On prépare une solution à 10 % (p/V) de gélatine (Bloom = 200, point isoélectrique 5) dans un tampon phosphate de pH donné.

A 35°C, on suspend 500 mg de micro-organismes dans cette solution et on ajoute un volume donné de solution aqueuse (concentration 10 % p/V) de dextran de masse moléculaire voisine de 70 000 dont le degré d'oxydation est de 10 %, 20 % ou 50 %. Le mélange est introduit à l'aide d'une seringue dans un récipient à long col, contenant du tampon phosphate pH 8, surmonté d'une colonne de décaline. La paroi des gouttelettes durcit au cours de leur chute dans le solvant organique et la masse réticule dans le tampon se trouvant en dessous. On isole des grains dont le diamètre est de 2,3 mm après la fin de la réticulation.

A pH 7 la réticulation n'est pas terminée, dans ces conditions, alors que, lorsqu'on mélange 6 volumes de solution de gélatine avec 1 volume de solution de dextran oxydé à 50 %, la réticulation est terminée en 2 heures à pH 10.

Les grains qui ne fondent pas à 60°C, et ont une bonne résistance mécanique, peuvent être conservés en solution aqueuse vers 5°C, de préférence en présence d'antibactérien (NaN$_3$ à 0,01 %).

## II - Efficacité des micro-organismes immobilisés

Cette bactérie transforme le saccharose en glucose-1-phosphate et fructose en présence de phosphate minéral.

La quantité de fructose formée peut être mesurée par chromatographie en phase gazeuse ou celle de glucose-1-phosphate par une méthode enzymatique mettant en oeuvre l'oxydation par NADP$^+$, en présence de glucose-6-phosphate-déshydrogénase, du glucose-6-phosphate formé par isomérisation du glucose-1-phosphate en présence de phosphoglucomutase. Le rapport des quantités des substrats formés à partir du saccharose, en mettant en oeuvre la même quantité de micro-organisme libre ou immobilisé, traduit la perte d'activité du micro-organisme, et est défini comme "l'efficacité" de la réaction d'immobilisation.

Dans les tableaux I et II, figurent les efficacités déterminées, en fonction du degré d'oxydation du dextran et de la durée de la réaction de réticulation pour un mélange 50/50 de gélatine et de dextran oxydé.

TABLEAU I

| DEGRE D'OXYDATION | PROPORTION DE GELATINE/ DEXTRAN OXY. | EFFICACITE |
|---|---|---|
| 10 % | 1/1 | 81,2 % |
| | 2/1 | 83,7 % |
| | 4/1 | 86,6 % |
| 20 % | 1/1 | 80,9 % |
| | 2/1 | 84,7 % |
| | 4/1 | 89,6 % |
| 50 % | 1/1 | 74,8 % |
| | 2/1 | 79,0 % |
| | 4/1 | 80,4 % |

TABLEAU II

| DEGRE D'OXYDATION | DUREE DE RETICULATION | EFFICACITE |
|---|---|---|
| 10 % | 2 | 82 |
| | 3 | 80 |
| | 4 | 74 |
| 20 % | 2 | 80 |
| | 3 | 78 |
| | 4 | 71 |
| 50 % | 2 | 75 |
| | 3 | 69 |
| | 4 | 58 |

## III - Utilisation de la composition de Leuconotoc mesenteroïdes immobilisés pour une réaction de fermentation en colonne

On introduit dans une colonne de hauteur 30 cm et de diamètre intérieur 1,5 cm, thermostatée à 15°C, 5 g de grains préparés comme décrit précédemment dans lesquels ont été immobilisés 100 mg ou 500 mg de bactéries, en suspension dans un tampon phosphate à pH 7. On fait circuler sur cette colonne une solution aqueuse de saccharose (0,5 M) à un débit de 1 ml/minute. La demi-vie des micro-organismes est de 270 heures, tandis qu'elle est de 281 heures lorsqu'ils sont en suspension.

Dans le tableau III ci-dessous, figurent les concentrations de fructose dans l'effluent à la sortie de la colonne.

TABLEAU III

| CARACTERISTIQUES DE LA COLONNE | DATE DU PRELEVE-MENT (EN HEURES) | CONCENTRA-TION DE FRUCTOSE mmol/l |
|---|---|---|
| 100 mg bactéries pour 5 g grains | 10 h | 2 |
| 500 mg bactéries pour 5 g grains | 10 h | 7 |

## EXEMPLE 3

Glucose isomérase immobilisée dans des grains à base de gélatine et de dextrane oxydé.

## I - Préparation d'une composition

Les grains sont préparés comme dans l'exemple 1-b, mais en remplaçant l'isooctane par de la décaline, ce qui donne des grains de forme plus régulière et bien stables.

On introduit respectivement 0,342 unité internationale, 0,684 unité internationale, 3,42 unités internationales et 34,24 unités internationales de glucose isomérase dans une solution de 0,2 g de gélatine de point isoélectrique 5 et 0,2 g de dextran, de masse moléculaire 70 000 environ oxydé à 20 %, dans 2 ml de tampon phosphate de pH = 7,5 et force ionique 0,2, contenant 3 mM de $MgSO_4$ ; on compare l'activité de la même quantité d'enzyme libre et immobilisée, en mesurant la quantité de glucose formée en fonction du temps dans une solution tampon de fructose à 75, %.

L'efficacité est fonction de la quantité d'enzyme immobilisée, elle est de 20 % environ lorsque peu d'enzyme est fixée (essais avec 0,342 à 3,42 unités internationales et de 45 % pour l'essai dans lequel 34,24 unités sont fixées).

Si l'on immobilise cette même enzyme selon une technique connue, comme celle décrite par R. Van Tilburg dans : Engineering Aspects of Biocatalysts in Industrial Starch Conversion Technology p. 27-43. Delftse Univ. Pers, (1983), l'efficacité est nettement inférieure, comme le montre l'essai suivant :

on dissout 0,32 g de gélatine, de point isoélectrique 5, dans 4 ml d'eau distillée et ajoute à cette solution 0,684u ou 34,2u d'enzyme ; le mélange est introduit sous forme de fines gouttelettes dans de l'acétate de butyle glacé ; les grains formés sont décantés au bout d'une heure, lavés à l'éthanol glacé et introduits dans une solution aqueuse de glutaraldéhyde à 2,5 (p/V) ; au bout de 3 heures les grains sont isolés, lavés à l'eau et l'activité enzymatique déteminée. Dans le premier essai (0,684u), l'efficacité calculée n'est que de 4,5 %, et dans le second de 1 %.

## EXEMPLE 4

Gélatine réticulée avec du xanthane oxydé.

### 1. Oxydation du polysaccharide:

On dissout 10 g de xanthane dépolymérisé (viscosité 0,25 Pa.s) dans 2 l d'eau distillée. On ajoute à cette solution, sous agitation, une solution aqueuse de periodate de sodium préparée à partir de 4,58 g de periodate dans 20 ml d'eau. Après 17 heures à température ambiante, à l'abri de la lumière le milieu réactionnel est transféré dans des membranes de dialyse. On dialyse pendant 2 jours contre l'eau du robinet et un jour contre l'eau distillée. On isole le polysaccharide oxydé par lyophilisation des solutions dialysées. Les fonctions alcools secondaires sont oxydées à 40 %, 25 % des fonctions aldéhydes formées sont libres (dosables par l'hydroxylamine).

### 2. Immobilisation de β-galactosidase :

A) préparation des grains:

On dissout d'une part 0,3 g de gélatine de point isoélectrique 5 et de Bloom 200 dans 2 ml de tampon phosphate à pH 8 (force ionique I = 0,05) et d'autre part 0,3 g de xanthane oxydé dans 8 ml de même tampon.

On mélange les 2 solutions à 40° C et on ajoute 2,40 mg d'enzyme (3,40 unités/mg) en solution dans 0,5 ml d'un tampon de pH 6,5. Le mélange est transféré dans une seringue et introduit sous forme de gouttelettes dans un récipient contenant 0,5 l d'une solution aqueuse tamponée à pH 8, surmontée sur une hauteur de 30 cm de 1l de décaline glacé ; les grains en suspension sont laissées 20 h sous agitation.

La taille des grains est fonction du diamètre de l'aiguille utilisée pour l'introduction ; pour un diamètre de l'aiguille de 0,9 mm on a obtenu 8,5 g de grains de diamètre 2,9 mm à l'état humide, ou 1,8 mm après séchage 48 h en présence de $P_2O_5$. Les grains séchés, remis en solution aqueuse, regonflent, mais, s'ils sont plus résistants, leur diamètre est inférieur (65%).

B) Etudes des grains :

### a) Importance et stabilité de l'immobilisation:

On a vérifié qu'il n'y avait pas d'enzyme restant dans les milieux de réticulation et que, lorsque les grains sont mis en solution aqueuse à pH 6,5, il n'y pas de libération de l'enzyme dans la solution après 1 heure.

Pour cela 4,2 g de grains isolés du milieu réactionnel, non séchés, sont introduits dans 20 ml de solution aqueuse ; après 1 h d'agitation, on prélève 2 ml de cette solution et on l'introduit dans 2 ml d'une solution à 7,5 % de lactose dans un tampon de pH 6,5 ("milk buffer"). Après 60 min. à 37°C, on prélève 200 μl de cette solution et on l'introduit dans 1,8 ml d'une solution $ZnSO_4$/$Na_2SO_4$(34mmol $ZnSO_4$ et 88 mmol $Na_2SO_4$ par litre), puis on ajoute 100 ul de NaOH 0,5 M ;
Le précipité est séparé par centrifugation, 0,5 ml du surnageant est introduit dans 2,5 ml de révélateur de glucose (2 mg glucose oxidase, 1mg peroxidase et 10 mg O-toluidine dans 150 ml de tampon acétate de pH 5). Après 8 min. à 20°C l'absorption est mesurée à 625 nm.

### b) Activité de l'enzyme immobilisée dans les grains, comparée à celle de la même quantité d'enzyme en solution

Les grains préparés comme précédemment sont divisés en 2 parties égales. Une partie est séchée puis réhydratée, l'autre est utilisée juste filtrée, chacune contient 4,08 unités enzymatiques.

Les grains ou la poudre d'enzyme sont introduits dans 15,4 ml d'une solution de lactose à 7,5 % (p/v) dans le tampon pH -6,5 "milk buffer" et on ajoute 7,7 ml du même tampon, pour obtenir une solution contenant 0,18 unités enzymatiques par ml. On prélève toutes les 15 minutes et pendant 2 heures un échantillon de ce mélange, maintenu à 37°C, et on mesure la quantité de glucose présent, en appliquant la méthode décrite dans l'essai mentionnée en a).

Les résultats obtenus figurent dans le tableau suivant ; l'efficacité de l'immobilisation est égale au rapport des absorptions mesurées dans les essais avec enzyme immobilisée et enzyme en solution.

| | heures de prélève- ment | absorp- tion | efficacité |
|---|---|---|---|
| enzyme en solution | 30 min. | 1,176 | 100 % |
| enzyme fixée (grains non séchés) | 30 min. | 0,102 | 9 % |
| enzyme fixée (grains séchés et réhydratés) | 30 min. | 0,077 | 6 % |

3 - Immobilisation de glucose isomérase.

a) Préparation des grains:

Les goutelettes sont préparées comme décrit dans 2 A. On introduit 110 unités de glucose isomérase (200 µl ; 550 µ/ml) dans un solution de 0,3 g de gélatine (point iso 5;200 Bloom) et 0,3 g xanthane dans 10 ml d'un tampon de phosphate pH 8 et on forme les grains comme précédemment.

On met les grains dans un tampon phosphate pH 7,5 (I = 0,2) contenant 3mM de $MgSO_4$. Après une heure, la moitié des grains (les autres sont séchés) est transférée dans 20 ml d'une solution à 7,5 % de fructose (dans un tampon pH 7,5 contenant du $MgSO_4$) et 10 ml de tampon. Le glucose formé est mesuré comme décrit précédemment.

L'efficacité de l'immobilisation est de 6 % environ pour les grains juste filtrés et de 5 % pour les grains séchés et réhydratés ; cette efficacité est le double de celle mesurée pour des grains réticulés au glutaraldéhyde.

**Revendications**

1. Composition d'enzymes, d'autres protéines ou de micro-organismes immobilisés, caractérisée en ce qu'elle est constituée d'un mélange de gélatine et du produit à immobiliser, qui a été réticulé par réaction avec un dextran ou un xanthane oxydé soluble dans l'eau.

2. Composition selon la revendication 1, caractérisée en ce que les produits immobilisés sont des micro-organismes.

3. Composition selon la revendication 1, caractérisée en ce que les produits immobilisés sont des enzymes.

4. Composition selon l'une quelconque des revendications 1 à 3, caractérisée en ce que le dextran à une masse moléculaire comprise entre 10 000 et 100 000.

5. Composition selon la revendication 4, caractérisée en ce que 10 à 75 % des unités glucose du dextran sont oxydées.

6. Composition selon l'une quelconque des revendications 1 à 5, caractérisée en ce qu'elle est sous forme de grains.

7. Procédé de préparation d'une composition selon l'une quelconque des revendications 1 à 6, caractérisé en ce que l'on fait réagir le dextran ou le xanthane sur un mélange de gélatine et du produit à immobiliser en milieu aqueux à un pH supérieur au point isoélectrique de la gélatine.

8. Procédé selon la revendication 7, caractérisé en ce que l'on fait réagir le dextrane ou le xanthane sur un mélange de gélatine de type A, et du produit à immobiliser en milieu aqueux à un pH compris entre 6 et 8.

Office européen
des brevets

**RAPPORT DE RECHERCHE EUROPEENNE**

Numero de la demande

EP 88 40 2322

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | DIE MAKROMOLEKULARE CHEMIE, vol. 182, no. 6, juin 1981, pages 1641-1648, DE; R.H. REINER: "HIO4-oxidezed soluble polysaccharides as polyfunctional links for covalent binding of enzymes, 1. Preparation of polysaccharides and matrices for their binding" * Page 1641; résumé * | 1-8 | C 12 N 11/10 |
| Y | PATENT ABSTRACTS OF JAPAN, vol. 7, no. 167 (C-177)[1312], 22 juillet 1983, page 127 C 177; JP-A-58 76 089 (SUMITOMO KAGAKU KOGYO K.K.) 09-05-1983 * Résumé * | 1-8 | |
| A | PATENT ABSTRACTS OF JAPAN, vol. 4, no. 26 (C-1)[508], 6 mars 1980, page 15 C 1; & JP-A-55 93 (SEISAN KAIHATSU KAGAKU KENKYUSHO) 05-01-1980 * Résumé * | 1-8 | |
| A | DD-A- 150 508 (AKADEMIE DER WISSENSCHAFTEN DER DDR) | | **DOMAINES TECHNIQUES RECHERCHES (Int. Cl.4)** C 12 N |
| A | CHEMICAL ABSTRACTS, vol. 104, no. 13, 31 mars 1986, page 47, résumé no. 102212d, Tokyo, JP; N. NOBUO et al.: "Immobilization of urease to modified dialdehyde-starch with gelatin for removal of urea", & KOBUNSHI RONBUNSHU 1985, 42(11), 835-9 | | |

Le présent rapport a été établi pour toutes les revendications

| Lieu de la recherche | Date d'achèvement de la recherche | Examinateur |
|---|---|---|
| LA HAYE | 12-12-1988 | FERNANDEZ Y BRANAS F.J. |